# EUROPÄISCHE PATENTSCHRIFT

(11) **EP 1 037 646 B1**
(45) Veröffentlichungstag und Bekanntmachung des Hinweises auf die Patenterteilung: **04.09.2002**
(21) Anmeldenummer: 98966583.1
(22) Anmeldetag: 18.12.1998
(51) Int. Cl.: A61K 35/78, A61P 25/00

(54) **EXTRAKTE AUS BLÄTTERN VON GINKGO BILOBA MIT VERMINDERTEM GEHALT AN 4'-O-METHYLPYRIDOXIN UND BIFLAVONEN**
GINKGO BILOBA LEAF EXTRACTS WITH A REDUCED 4'-O-METHYLPYRIDOXINE AND BIFLAVONE CONTENT
EXTRAITS DE FEUILLES DE GINKGO BILOBA PRESENTANT UNE TENEUR REDUITE EN 4'-O-METHYLPYRIDOXINE ET EN BIFLAVONES

(30) Priorität: 19.12.1997 DE 19756848
(43) Veröffentlichungstag der Anmeldung: 27.09.2000
(73) Patentinhaber: Dr. Willmar Schwabe GmbH & Co., 76227 Karlsruhe (DE)
(72) Erfinder: SCHWABE, Klaus-Peter, D-76227 Karlsruhe (DE)
(74) Vertreter: Banzer, Hans-Jörg, Dipl.-Ing.
(86) Internationale Anmeldenummer: DE9803790
(87) Internationale Veröffentlichungsnummer: WO99032129

(56) Entgegenhaltungen:
- EP-A- 0 330 567
- EP-A- 0 431 535

## Beschreibung

Die Erfindung betrifft Extrakte aus Blättern von Ginkgo biloba mit vermindertem Gehalt an 4'-O-Methylpyridoxin und Biflavonen, ein Verfahren zu ihrer Herstellung und diese Extrakte enthaltende Arzneimittel, die selbst bei hoher Dosierung keine unerwünschten Nebenwirkungen zeigen, die durch 4'-O-Methylpyridoxin und Biflavone ausgelöst werden.

Extrakte aus den Blättern von Ginkgo biloba werden seit 30 Jahren therapeutisch verwendet. Die Kommission E beim Bundesgesundheitsamt in Berlin hat Arzneimittel mit definierten Extrakten als wirksam für die symptomatische Behandlung von hirnorganisch bedingten Leistungsstörungen, bei peripherer arterieller Verschlußkrankheit, Schwindel und Tinnitus anerkannt (BAnz Nr. 133 vom 19.Juli 1994). Diese Extrakte sind charakterisiert durch einen Gehalt an 22 bis 27 % Flavonglykosiden, 5 bis 7 % Terpenlaktonen, davon 2,8 bis 3,4 % Ginkgolide A, B und C sowie 2,6 bis 3,2 % Bilobalid und einen Maximalgehalt von 5 ppm Ginkgolsäuren (Alkylphenol-Verbindungen). Solche Extrakte aus Blättern von Ginkgo biloba sowie Verfahren zu ihrer Herstellung sind bekannt aus der DE-PS 39 40 091 (EP-B-0 431 535) und DE-PS 39 40 092 (EP-B-0 431 536).

Das Vorkommen von 4'-Methoxypyridoxin (4'-O-Methylpyridoxin), in den Samenkernen von Ginkgo biloba wurde von Wada et al.,Chem. Pharm. Bull. 33 (1985), 3555-3557 beschrieben. Diese Verbindung verursacht Vergiftungserscheinungen wie Krampfanfälle und Bewußtlosigkeit ("Gin-nan food poisoning" bzw. "Gin-nan sitotoxism"), die auftreten können nach dem Verzehr von größeren Mengen an Ginkgo Samenkernen. Die Entdecker dieser Substanz haben diese deshalb als "Ginkgotoxin" bezeichnet. In einer späteren Arbeit hat die gleiche Forschergruppe das Fehlen von 4'-O-Methylpyridoxin in Ginkgo Blättern festgestellt (Wada et al., Biol. Pharm. Bull. 16 (1993), 210-212. Im Gegensatz zu dieser Aussage steht eine erst kürzlich erfolgte Publikation, in welcher über die Isolierung und Identifizierung von 4'-O-Methylpyridoxin aus Ginkgo Blättern berichtet wurde (Arenz et al., Planta Medica 62 (1996), 548-551). In dieser Arbeit wurden Handelspräparate mit Extrakten aus Blättern von Ginkgo biloba auf den Gehalt an Antivitamin B₆ (4'-O-Methylpyridoxin) untersucht und Konzentrationen zwischen ca. 4 und 10 µg pro ml flüssiger Arzneiform gefunden. Dies entspricht einer Konzentration von ca. 100 bis 250 ppm in den zu Präparaten verarbeiteten Trockenextrakten.

Die in der Therapie verwendeten Ginkgo-Extrakte enthalten außerdem bis zu 1000 ppm Biflavone. In eigenen Untersuchungen hat es sich überraschend gezeigt, daß diese Verbindungen zusätzlich zu den literaturbekannten biologischen Wirkungen immuntoxikologische Effekte besitzen. Im Popliteallymphknoten-Assay an der Maus wurde von uns beobachtet, daß die in Ethylacetat lösliche Fraktion eines Äthanol-Wasser-Gesamtextraktes aus Ginkgo-Blättern eine Lymphknotenreaktion hervorrufen, vgl. Beispiel 1. Testung der durch chromatographische Auftrennung erhaltenen Unterfraktionen ergab, daß der immuntoxikologische Effekt nur nach Applikation der Unterfraktion auftrat, welche über 70 % Biflavone enthielt. Bei den Biflavonen in Ginkgo handelt es sich um folgende Verbindungen: Sciadopitysin, Ginkgetin, Isoginkgetin, Sequoiaflavon und Bilobetin.

Aufgabe der vorliegenden Erfindung ist es, einen Extrakt aus den Blättern von Ginkgo biloba bereitzustellen, der praktisch frei von 4'-O-Methylpyridoxin und Alkylphenol-Verbindungen (Ginkgolsäuren, Ginkgol) ist, den technisch machbaren Minimalgehalt an Biflavonen und die als wirksamkeitsbestimmenden Inhaltsstoffe anerkannten Flavonolglykoside und Terpenlaktone in den von der Kommission E (BAnz Nr. 133 vom 19. Juli 1994) geforderten Konzentrationen aufweist. Der erfindungsgemäße Extrakt enthält:
- 20 bis 30 Gew.% Flavonolglykoside
- 2,5 bis 4,5 Gew. % insgesamt der Ginkgolide A,B,C und J
- 2,0 bis 4,0 Gew.% Bilobalid
- weniger als 10 ppm, insbesondere weniger als 1 ppm Alkylphenol-Verbindungen (Ginkgolsäuren, Ginkgol)
- weniger als 10 Gew. % Proanthocyanidine
- weniger als 50 ppm 4'-O-Methylpyridoxin
- weniger als 100 ppm Biflavone

Die Erfindung betrifft ferner ein Verfahren zur Herstellung eines solchen Extraktes aus Blättern von Ginkgo biloba. Diese Aufgabe wird dadurch gelöst, daß bei den bekannten Herstellungsverfahren für Ginkgo-Extrakte zwei zusätzliche Verfahrensschritte eingeführt werden:
1. die Behandlung mit Kationenaustauscher zur Entfernung des 4°-O-Methylpyridoxins
2. die Adsorption der Biflavone an Aktivkohle.

Diese Maßnahmen werden im Herstellungprozeß nach der DE-PS 39 40 091 durchgeführt beim Verfahren nach Anspruch 3 vorzugsweise vor oder nach der Stufe g). So wird nach dem Filtrieren der in Stufe f) erhaltenen Lösung diese über eine Kationenaustauscher-Säule gegeben und die Säule mit wäßrigem Ethanol oder wäßrigem Methanol eluiert. Das erhaltene Eluat wird mit Aktivkohle behandelt. Zum Eluieren des Kationenaustauschers kann eine wäßrige Lösung mit 20 bis 80 Gew.-% vorzugsweise 50 Gew.-% Ethanol oder Methanol verwendet werden. Die Reihenfolge dieser beiden erfindungsgemäßen Schritte kann vertauscht werden ohne Änderung des Ergebnisses. Danach wird wie nachstehend in Beispiel 3 beschrieben weiter gearbeitet und die Lösung mit einem aliphatischen oder cycloaliphatischen Lösungsmittel zur Entfernung der Alkylphenol-Verbindungen extrahiert.

Der Verfahrensschritt c) dient zur Entfernung der in Wasser schwer löslichen lipophilen Bestandteile, einschließlich der Hauptmenge an Biflavonen.

Das bevorzugte Verfahren der Erfindung zur Herstellung eines Extraktes nach Anspruch 1 ist demnach gekennzeichnet durch die Anzahl und Reihenfolge der Verfahrensstufen:
a) Extraktion von Blättern von Ginkgo biloba mit wasserhaltigem Aceton, einem wasserhaltigen Alkanol mit 1 bis 3 C-Atomen oder wasserfreiem Methanol,
b) Abtrennen des organischen Lösungsmittels durch Einengen des Lösungsmittels auf einen Gehalt von höchstens 10 Gew.-%, wobei den letzten Destillationsstufen Wasser zugesetzt werden kann,
c) Verdünnen der verbleibenden wäßrigen Lösung mit Wasser auf einen Feststoffgehalt von 5 bis 25 Gew.-%, Abkühlen auf eine Temperatur bis unter 25°C und Stehenlassen bis zur Ausbildung eines Niederschlags,
d) eine Behandlung der verbleibenden wäßrigen Lösung mit Ammoniumsulfat und darauffolgend mindestens eine Extraktion mit Methylethylketon oder einem Gemisch aus Methylethylketon und Aceton,
e) Konzentrieren des erhaltenen Extraktes und Verdünnen mit einem Ethanol-Wasser-Gemisch auf eine Lösung, enthaltend je 50 Gew.-% Wasser und Ethanol mit 10 Gew.-% Feststoffgehalt,
f) Behandlung der Lösung mit einer Bleiverbindung oder einem unlöslichen Polyamid,
g) Filtration der Lösung und Passage über einen stark sauren Kationenaustauscher, Elution des Kationenaustauschers mit wäßrigem Ethanol oder wäßrigem Methanol und
h) Behandlung des Eluats mit Aktivkohle,
i) Extraktion der filtrierten Lösung mit einem aliphatischen oder cycloaliphatischen Lösungsmittel mit einem Siedepunkt von 60 bis 100°C;
j) Konzentrieren der verbleibenden wäßrig-alkoholischen Lösung anschließend Behandlung mit Ammoniumsulfat und Extraktion mit Methylethylketon und Ethanol,
k) Konzentrieren der erhaltenen organischen Phase auf einen Feststoffgehalt von 50 bis 70 Gew.-%, und
l) Trocknen des Konzentrates unter vermindertem Druck zu einem Trockenextrakt mit einem Wassergehalt von weniger als 5%.

In einer weiteren Ausführungsform der Erfindung sind die Verfahrensschritte g) und h) vertauscht.

In einer noch weiteren Ausführungsform der Erfindung werden die Verfahrensschritte g) und h) erst nach dem Schritt i) ausgeführt.

Bei den bekannten Verfahren zur Anreicherung der Flavonolglykoside und Terpenlaktone über Adsorptionsharze (EP-B-0 360 556, JP-A-04182434) werden die beiden erfindungsgemäßen Schritte mit der methanolisch-wäßrigen Lösung des Extraktes aus dem Desorptionschritt (vgl. EP-B-0 360 556, Beispiel III, Anspruch 12) bzw. der ethanolischwäßrigen Lösung nach JP-A-0 418 2434 durchgeführt.

Beispiele für verwendbare Kationenaustauscher sind saure Kationenaustauscher. Der besonders bevorzugte Kationenaustauscher ist ein stark saurer Kationenaustauscher wie Merck I.

Der erfindungsgemäß hergestellte Extrakt zeigt in pharmakologischen Versuchsmodellen durchblutungsfördernde, Ischämieschäden verhindernde, Radikalfänger- und die Thrombozytenaggregation hemmende Eigenschaften, wobei im Gegensatz zu den bisher bekannten Ginkgo-Extrakten selbst bei hoher Dosierung keine unerwünschte Effekte auftreten können, die durch 4'-O-Methylpyridoxin und Biflavone ausgelöst werden.

Die Erfindung betrifft außerdem Arzneimittel, die durch einen Gehalt an Ginkgo biloba Extrakt gemäß vorliegender Erfindung gekennzeichnet sind.

Zur Herstellung von Arzneimitteln kann der erfindungsgemäße Extrakt in üblicher Weise verarbeitet werden, z. B. zu Lösungen, Dragees, Tabletten oder Injektionspräparaten. Die Arzneimittel werden zur Behandlung von peripheren und cerebralen arteriellen Durchblutungsstörungen verwendet.

### Beispiel 1

Popliteallymphknoten-Test: Es wurden männliche C57BL/6-Mäuse von ca. 18-24 g Gewicht verwendet (Charles River, Sulzfeld). Die Haltung der Tiere erfolgte unter standardisierten Umweltbedingungen bei freiem Zugang zu Futter und Wasser. Zur Induktion der Popliteallymphknoten-Reaktion (PLR) wurden die Testsubstanzen in 10 µl DMSO subplantar in die rechte Hinterpfote injiziert. Die Tiere in der Kontrollgruppe erhielten nur eine Injektion von 10 µl DMSO. Von den Einzelextrakten wurden immer 2 mg verabreicht. Die Dosis für die verschiedenen Unterfraktionen entsprach jeweils ihrem Anteil am Ausgangsextrakt. Sieben Tage später wurden die Versuchstiere in Äthernarkose durch zervikale Dislokation getötet. Beide Popliteallymphknoten wurden entnommen und nach Entfernung von anhaftendem Gewebe auf angefeuchtetem Filterpapier in Petrischalen gesammelt. Unmittelbar anschließend wurde das Gewicht der Lymphknoten auf einer elektronischen Waage mit einer Genauigkeit von 0,1 mg bestimmt. Die Stärke der PLR ist jeweils als die Gewichtsdifferenz zwischen dem ipsi- und dem contralateralen Popliteallymphknoten angegeben.

| **Substanz** | **Dosis** | **Lymphknotengewicht (mg)** |
|---|---|---|
| Ethylacetatfraktion | 2,00 mg | 4,2 mg |
| Biflavonfraktion | 0,35 mg | 3,4 mg |

### Beispiel 2

250 ml Überstand aus der Blei-Gerbstoff-Fällung nach Beispiel 1 der DE-PS 39 40 091 (Spalte 4, Zeile 67) , enthaltend 10 % Trockenrückstand in der Lösung mit 50 Gew. % Ethanol, werden mit 2,5 g Aktivkohle versetzt. Dieses Gemisch wird 30 Minuten bei Raumtemperatur gerührt. Die Aktivkohle wird abfiltriert und das Filtrat über eine Säule mit 20 ml stark saurem Kationenaustauscher (Merck I) gegeben. Die eluierte Lösung wird gemäß dem in Beispiel 1 der DE-PS 39 40 091 beschriebenen Verfahren weiter aufgearbeitet.

Nach Trocknung der Lösung des Endprodukts in Methyläthylketon/Ethanol werden erhalten 22,25 g Trockenextrakt mit einem Gehalt von 23,9 % Ginkgoflavonglykosiden, 3,3 % Ginkgoliden, 2,9 % Bilobalid, 7,1 % Proanthocyanidinen, weniger als 1 ppm Alkylphenöl-Verbindungen, weniger als 50 ppm^{*)} 4'-O-Methylpyridoxin und 60 ppm Biflavonen ^{**)}.
^{*)} Analysenmethode nach Arenz et al., *Planta Medica*, Bd. 62 (1996), 548-551
^{**)} Analysenmethode nach Gobbata et al., *Fitoterapia,* Bd. 67 (1996), 152-158

### Beispiel 3

250 ml 90% methanolisch-wäßrige Lösung aus der Desorption der Flavone und Terpenlactone von Adsorberharz Duolite S-761 nach Beispiel III, Seite 5, Zeile 41 der EP-B-0 360 556, enthaltend 10% Trockenrückstand in 90 Gew. % Methanol, wird über eine Säule mit 15 ml stark saurem Kationenaustauscher filtriert. Mit 3,75 g Aktivkohle wird diese Lösung unter vermindertem Druck von einem Teil des Methanols befreit und durch Zugabe von Wasser auf eine Methanolkonzentration von 50 Gew.-% eingestellt und 30 Minuten bei Raumtemperatur gerührt. Die Aktivkohle wird über eine Filterschicht abgesaugt und mit 20 ml 50 % Methanol nachgewaschen. Nach dem Einengen der Lösungen unter vermindertem Druck zur Trockne verbleiben 23,2 g Ginkgo-Extrakt mit einem Gehalt von 22,8 % Ginkgoflavonglykosiden, 3,1% Ginkgoliden, 2,8 % Bilobalid, 9,6% Proanthocyanidinen, weniger als 1 ppm Alkylphenol-Verbindungen, weniger als 50 ppm 4'-O-Methylpyridoxin und 28 ppm Biflavonen.

### Beispiel 4

Rezeptur für Ginkgo-Extrakt Tabletten:

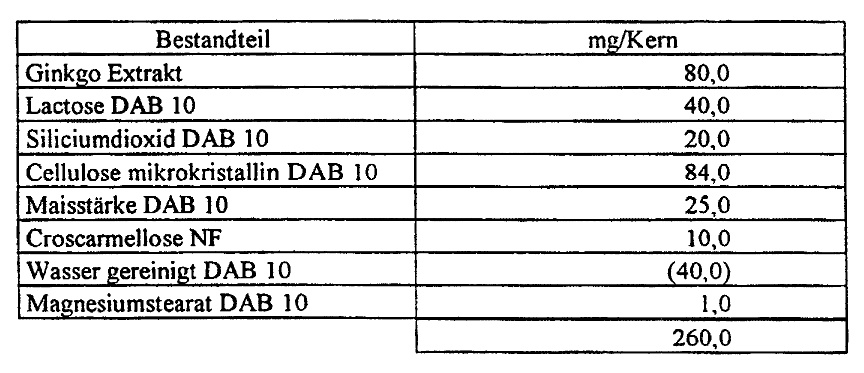

Die Bestandteile werden gemischt, mit Wasser nach üblichen Verfahren im Mischer oder Wirbelsprühgerät granuliert und anschließend auf einer Rundlauf-Tablettenpresse tablettiert.

## Patentansprüche

1. Extrakt aus den Blättern von Ginkgo biloba, enthaltend
- 20 bis 30 Gew.% Flavonolglykoside
- 2,5 bis 4,5 Gew. % insgesamt der Ginkgolide A,B,C und J
- 2,0 bis 4,0 Gew.% Bilobalid
- weniger als 10 ppm Alkylphenol-Verbindungen
- weniger als 10 Gew. % Proanthocyanidine
- weniger als 50 ppm 4'-O-Methylpyridoxin
- weniger als 100 ppm Biflavone.

2. Extrakt nach Anspruch 1, wobei der Extrakt weniger als 1 ppm Alkylphenol-Verbindungen enthält.

3. Verfahren zur Herstellung eines Extraktes nach Anspruch 1 oder 2, **gekennzeichnet durch** die Anzahl und Reihenfolge der Verfahrensstufen:
a) Extraktion von Blättern von Ginkgo biloba mit wasserhaltigem Aceton, einem wasserhaltigen Alkanol mit 1 bis 3 C-Atomen oder wasserfreiem Methanol,
b) Abtrennen des organischen Lösungsmittels **durch** Einengen des Lösungsmittels auf einen Gehalt von höchstens 10 Gew.-%, wobei den letzten Destillationsstufen Wasser zugesetzt werden kann,
c) Verdünnen der verbleibenden wäßrigen Lösung mit Wasser auf einen Feststoffgehalt von 5 bis 25 Gew.-%, Abkühlen auf eine Temperatur bis unter 25°C und Stehenlassen bis zur Ausbildung eines Niederschlags,
d) eine Behandlung der verbleibenden wäßrigen Lösung mit Ammoniumsulfat und darauffolgend mindestens eine Extraktion mit Methylethylketon oder einem Gemisch aus Methylethylketon und Aceton,
e) Konzentrieren des erhaltenen Extraktes und Verdünnen mit einem Ethanol-Wasser-Gemisch auf eine Lösung, enthaltend je 50 Gew.-% Wasser und Ethanol mit 10 Gew.-% Feststoffgehalt,
f) Behandlung der Lösung mit einer Bleiverbindung oder einem unlöslichen Polyamid,
g) Filtration der Lösung und Passage über einen stark sauren Kationenaustauscher, Elution des Kationenaustauschers mit wäßrigem Ethanol oder wäßrigem Methanol und
h) Behandlung des Eluats mit Aktivkohle,
i) Extraktion der filtrierten Lösung mit einem aliphatischen ode cycloaliphatischen Lösungsmittel mit einem Siedepunkt von 60 bis 100°C;
j) Konzentrieren der verbleibenden wäßrig-alkoholischen Lösung anschließen Behandlung mit Ammoniumsulfat und Extraktion mit Methylethylketon un Ethanol,
k) Konzentrieren der erhaltenen organischen Phase auf einen Feststoffgehalt von 5 bis 70 Gew.-%, und
l) Trocknen des Konzentrates unter vermindertem Druck zu einem Trockenextral mit einem Wassergehalt von weniger als 5%.

4. Verfahren nach Anspruch 3, wobei die Schritte g) und h) vertauscht sind.

5. Verfahren nach Anspruch 3 oder 4, wobei die Schritte g) und h) erst nach der Schritt i) ausgeführt werden.

6. Arzneimittel, **gekennzeichnet durch** einen Gehalt an Ginkgo biloba Extrakt nac Anspruch 1 oder 2.

7. Verwendung des Extraktes nach Anspruch 1 oder 2 zur Herstellung einer pharmazeutisch Zubereitung zur Behandlung von hirnorganisch bedingten Leistungsstörunge peripherer arterieller Verschlußkrankheit, Schwindel und Tinnitus.

## Claims

1. Extract from ginkgo biloba leaves, containing
- 20 to 30 % by weight flavonol glycosides
- a total of 2.5 to 4.5 % by weight of ginkgolides A, B, C and J
- 2.0 to 4.0 % by weight bilobalide
- below 10 ppm, alkyl phenol compounds
- below 10 % by weight proanthocyanidins
- below 50 ppm 4'-O-methyl pyridoxine
- below 100 ppm biflavones

2. Extract according to claim 1, containing below 1ppm alkyl phenol compounds.

3. Method for the preparation of an extract according to claim 1 or 2, **characterized by** the number and order of the following steps:
a) extraction of ginkgo biloba leaves with aqueous acetone, an aqueous alkanol having 1 to 3 carbon atoms or absolute methanol,
b) separating the organic solvent by concentrating the solvent up to a content of not more than 10 % by weight, wherein water can be added in the last distillation steps,
c) diluting the remaining aqueous solution with water to a content of solids of 5 to 25 % by weight, cooling to a temperature of below 25°C and allowing it to stand until a precipitate forms,
d) a treatment of the remaining aqueous solution with ammonium sulfate and thereafter at least one extraction with methyl ethyl ketone or a mixture of methyl ethyl ketone and acetone,
e) concentrating the obtained extract and diluting with an ethanol-water mixture to a solution containing each 50 % by weight water and ethanol and 10 % by weight of solids,
f) treatment of the solution with a lead compound or an insoluble polyamide,
g) filtration of the solution and passage through a strongly acidic cation exchanger, elution of the cation exchanger with aqueous ethanol or aqueous methanol,
h) treating the eluate with activated charcoal,
i) extraction of the filtered solution with an aliphatic or cycloaliphatic solvent having a boiling point of 60 to 100°C,
j) concentrating the remaining aqueous-alcoholic solution, subsequently treating with ammonium sulfate and extraction with methyl ethyl ketone and ethanol,
k) concentrating the obtained organic phase to a content of solids of 50 to 70 % by weight, and
l) drying the concentrate under vacuum to a dry extract having a water content of below 5 %.

4. Method according to claim 3, wherein steps g) and h) are interchanged.

5. Method according to claim 3 or 4, wherein steps g) and h) are performed only after step i).

6. Medicament, **characterized by** a content of ginkgo biloba extract according to claim 1 or 2.

7. Use of the extract according to claim 1 or 2 for the preparation of a pharmaceutical formulation for the treatment of performance disturbances caused by cerebral insufficiency, peripheral occlusive disease, vertigo and tinnitus.

## Revendications

1. Extrait de feuilles de Ginkgo biloba, contenant
- 20 à 30 % en poids de glycosides de flavonol
- 2,5 à 4,5 % en poids du total des ginkgolides A, B, C et J
- 2,0 à 4,0 % en poids de bilobalide
- moins de 10 ppm de composés d'alkylphénol
- moins de 10 % en poids de proanthocyanidines
- moins de 50 ppm de 4'-O-méthylpyridoxine
- moins de 10 ppm de biflavones.

2. Extrait selon la revendication 1, dans lequel l'extrait contient moins de 1 ppm de composés d'alkylphénol.

3. Procédé pour la préparation d'un extrait selon la revendication 1 ou 2, **caractérisé par** le nombre et la succession des étapes opératoires:
a) extraction de feuilles de Ginkgo biloba avec de l'acétone contenant de l'eau, un alcanol contenant de l'eau ayant 1 à 3 atomes de carbone ou du méthanol anhydre,
b) séparation du solvant organique par concentration du solvant jusqu'à une teneur d'au plus 10 % en poids, tandis que de l'eau peut être ajoutée dans les dernières étapes de distillation,
c) dilution de la solution aqueuse restante avec de l'eau jusqu'à une teneur en solides de 5 à 25 % en poids, refroidissement jusqu'à une température inférieure à 25°C et mise au repos jusqu'à formation d'un précipité,
d) un traitement de la solution aqueuse restante avec du sulfate d'ammonium et subséquemment au moins une extraction avec de la méthyléthylcétone ou un mélange de méthyléthylcétone et d'acétone,
e) concentration de l'extrait obtenu et dilution avec un mélange éthanol-eau pour former une solution contenant 50 % en poids respectivement d'eau et d'éthanol avec 10 % en poids de teneur en solides,
f) traitement de la solution avec un composé de plomb ou un polyamide insoluble,
g) filtration de la solution et passage sur un échangeur de cations fortement acide, élution de l'échangeur de cations avec de l'éthanol aqueux ou du méthanol aqueux et
h) traitement de l'éluat avec du charbon actif,
i) extraction de la solution filtrée avec un solvant aliphatique ou cycloaliphatique ayant un point d'ébullition de 60 à 100°C,
j) concentration de la solution aqueuse-alcoolique restant après traitement avec du sulfate d'ammonium et extraction avec de la méthyléthylcétone et de l'éthanol,
k) concentration de la phase organique obtenue jusqu'à une teneur en solides de 50 à 70 % en poids, et
l) séchage du concentré sous pression réduite jusqu'à un extrait sec ayant une teneur en eau inférieure à 5 %.

4. Procédé selon la revendication 3, dans lequel les étapes g) et h) sont inversées.

5. Procédé selon la revendication 3 ou 4, dans lequel les étapes g) et h) sont mises en oeuvre seulement après l'étape i).

6. Médicament, **caractérisé par** une teneur en extrait de Ginkgo biloba selon la revendication 1 ou 2.

7. Utilisation de l'extrait selon la revendication 1 ou 2 pour l'obtention d'une préparation pharmaceutique pour le traitement de troubles de la puissance à conditionnement organique par le cerveau, d'obstructions artérielles périphériques, de vertiges et de bourdonnement d'oreilles.
